# EUROPEAN PATENT SPECIFICATION

(11) **EP 3 236 978 B1**
(45) Date of publication and mention of the grant of the patent: **14.06.2023**
(21) Application number: 15816212.3
(22) Date of filing: 15.12.2015
(51) Int. Cl.: A61K 36/8962, A61K 31/352, A61K 31/255, A61K 31/194, A61K 31/105, A61K 31/10, A61K 31/7048, A61P 31/04, A61P 31/10, A61P 33/00, A61P 43/00

(54) **AN ANTIMICROBIAL COMPOSITION**
ANTIMIKROBIELLE ZUSAMMENSETZUNG
COMPOSITION ANTIMICROBIENNE

(30) Priority: 23.12.2014 GB 201423115
(43) Date of publication of application: 01.11.2017
(73) Proprietor: Mootral SA, 1180 Rolle (CH)
(72) Inventor: GRAZ, Carl Jorg Michael, Abertillery Blaenau Gwent NP13 1SX (GB); EVANS, Gareth James Street, Abertillery Blaenau Gwent NP13 1SX (GB); SAUNDERS, Robert Alun, Abertillery Blaenau Gwent NP13 1SX (GB); JONES, Liam James, Abertillery Blaenau Gwent NP13 1SX (GB)
(74) Representative: Haseltine Lake Kempner LLP
(86) International application number: PCT/GB2015/054018
(87) International publication number: WO 2016/102931

(56) References cited:
- EP-A1- 1 512 409
- EP-A1- 1 911 455
- EP-A1- 2 198 862
- WO-A2-2009/106890
- US-A1- 2013 123 207
- US-A1- 2013 224 281
- US-A1- 2014 127 179
- MERIGA BALAJI ET AL: "Insecticidal, antimicrobial and antioxidant activities of bulb extracts of Allium sativum", ASIAN PACIFIC JOURNAL OF TROPICAL MEDICINE, vol. 5, no. 5, May 2012 (2012-05), pages 391-395, XP55252134, ISSN: 1995-7645
- YUSUF O K ET AL: "Studies of phytochemical constituents and antitrypanosomal properties of fermented wheat germ and garlic bulbs extract on Trypanosoma brucei - infected rats", JOURNAL OF MEDICINAL PLANT RESEARCH 2010 ACADEMIC JOURNALS NGA, vol. 4, no. 19, 2010, pages 2016-2020, XP9188629, ISSN: 1996-0875

## Description

### Field of Invention

The present invention relates to antimicrobial compositions, as well as to uses of the same and methods for treating or preventing bacterial, fungal or parasitic infections.

### Background to the Invention

The worldwide problem of bacterial resistance has grown to such an extent that several organisms (for example, *Klebsiella pneumoniae, Escherichia coli and Staphylococcus aureus*) have developed resistance to multiple different antibiotics. According to the Centres for Disease Control and Prevention (CDC), in 2013 there were over 2 million infections with antibiotic resistant microorganisms and over 23 thousand subsequent deaths from those infections. A little under half of those deaths (11,000) occurred as a result of methicillin-resistant *S*. *aureus* (MRSA). Another organism that was responsible for a large number of deaths, (14,000, even higher than MRSA) in 2013, was *Clostridum difficile.* This bacterium has proven to be very resistant to antibiotics. However, the resistance of *C*. *difficile* is due to its ability to produce spores, rather than specific strain resistance (hence not being included in the year total for antibiotic resistance infection deaths).

Combinations of antimicrobials with improved efficacy are much sought after in the present clinical industry. The combination antibiotic therapy is used in an attempt to broaden the bacterial spectral range, and thus to avoid the emergence of resistance or multi-resistance and lead to a better clinical outcome. Understanding of the mechanisms of antimicrobial actions and researches on potential antimicrobial agents are vital in the development of an effective combination of antimicrobials.

US 2013/0224281 discloses compositions intended to protect against the effects of radiation and/or oxidative stress. The compositions contain, *inter alia,* quercetin (a bioflavonoid), garlic extract/allicin and alpha lipoic acid (an organic acid), and are described as a "daily dose of a useful mixture of antioxidant and chemoprotective agent mixtures for use with radiation and oxidative treatment compositions".

US 2013/0123207 describes resveratrol-containing compositions intended to provide "a therapeutic benefit to a subject such as modulation of a biological activity, improving cell transplantation therapy or improving macular degeneration or dystrophy effects". The compositions may additionally comprise antioxidants selected from a list including quercetin (a bioflavonoid) and ferulic acid (an organic acid).

US 2014/0127179 discloses a supplement intended to enhance natural killer cells in the body. The supplement comprises, *inter alia,* garlic, genistein (a bioflavonoid) and vitamin C (an organic acid) and is intended to treat cancer and/or viral infections.

EP 1911455 discloses a composition comprising, *inter alia,* lactic acid and a garlic extract, which is intended to restore and stabilise vaginal pH in order to reduce the risk of bacterial, fungal and mycoplasma infections.

EP 1512409 discloses a composition containing, *inter alia,* garlic and acetic acid, which is intended to treat head lice.

WO 2009/106890 and EP2198862 teach or suggest antimicrobial properties of compositions comprising bioflavonoids and organic acids. Meriga et al. (Asian Pacific Journal of Tropical Medicine, 2012, 5:391-395) and Yusuf (Journal of Medicinal Plant Research 2010 Academic Journals NGA, 4:2016-2020) teach or suggest antimicrobial properties of garlic or garlic extracts.

However, none of the above listed documents explicitly disclose a composition comprising a combination of a garlic extract, a bioflavonoid and/or an organic acid for use in treating or preventing a bacterial or fungal infection.

In view of the growing problem of antibiotic resistance, there is a need for novel antibiotic compositions that are effective against a wide variety of microorganisms, especially antibiotic resistant microorganisms.

### Summary of Invention

The present invention provides a novel combination of garlic compounds with bioflavonoids and/or organic acids, which can provide an antimicrobial effect, preferably a synergistic antimicrobial effect, against broad-spectrum of microorganisms, including bacteria, fungi and parasites.

The present invention provides an antimicrobial composition comprising at least one garlic extract and at least one bioflavonoid and optionally an organic acid for use in treating or preventing a *Staphylococcus aureus* or *Candida tropicalis* infection, wherein the garlic extract is selected from allicin and ajoene, wherein the bioflavonoid is selected from one or more of the group consisting of naringenin, naringin, quercetin and rhoifolin, and wherein the organic acid is a carboxylic acid.. The composition may also include at least one garlic extract, at least one bioflavonoid and at least one organic acid.

The concentration ratio of the garlic extract to the bioflavonoid can be about 1-16:16-1. For example, the concentration ratio of the garlic extract to the bioflavonoid is about 1-4:4-1. Similarly, the concentration ratio of the garlic extract to the organic acid can be about 1-16:16-1, for example, the concentration ratio of the garlic extract to the organic acid is about 1-4:4-1. For a composition comprises at least one garlic extract, at least one bioflavonoid and at least one organic acid, the concentration ratio of the garlic extract to the bioflavonoid and to the organic acid can be about 1-16:1-16:1-16. Alternatively, the concentration ratio of the garlic extract to the bioflavonoid and to the organic acid can be about 1-4:1-4:1-4.

The garlic extract can be selected from one or more of allicin and ajoene. The bioflavonoids can be one or more of rhoifolin quercetin, naringenin and naringin or any combination thereof. The organic acid used in the present invention is a carboxylic acid, such as lactic acid, butyric acid, propionic acid, valeric acid, caproic acid, acetic acid, formic acid, citric acid, oxalic acid, sorbic acid, benzoic acid, caprylic acid and malic acid, or any combination thereof.

The present invention provides an antimicrobial composition which may further comprise a pharmaceutically acceptable carrier and/or excipient. The composition can be formulated for oral, topical, intravenous, intramuscular, intrarectal (suppository), inhalant, infusion, transdermal, sublingual, subcutaneous or intranasal administration.

The antimicrobial composition may be an antibacterial composition, an antifungal composition or an antiparasitic composition. The composition may be used to treat or prevent bacterial infections such as Gram-negative or Gram-positive bacterial infections, fungal infections, or parasitic infections, as appropriate.

### Detailed Description

The combination of garlic extract with at least one bioflavonoid and optionally an organic acid as provided in compositions of the present invention has been found to produce a surprising and unexpected synergistic action against pathogenic microorganisms. This synergistic effect of compositions of the present invention has been determined based on the antimicrobial activity of the compositions. In particular, synergy has been determined where the antimicrobial activity of a composition containing a mixture of two or more components is higher than the combined antimicrobial activity of each component alone.

Without being bound by theory, it is believed that the modes of antimicrobial action of each component underlie the antimicrobial synergy observed when the components are combined. For example, garlic compounds such as allicin have an oxidising effect on the surface of microbes. Ajoene, however, acts intracellularly by affecting enzyme function. Bioflavonoids are taken up across cell membranes and act intracellularly. Bioflavonoids are known to have enzyme-inhibiting activity, they inhibit energy metabolism and it is further postulated that they cause damage to cell membranes, leading to the inhibition of macromolecules.

Organic acids, on the other hand, are able to cross cell membranes, where they alter intracellular pH. For instance, formic acid inhibits enzymatic activity, especially that of decarboxylase; acetic acid inhibits enzymatic activity and increases heat sensitivity; propionic acid influences membrane transport inhibition on synthesis of some amino acids; lactic acid inhibits enzymatic activity; sorbic acid and benzoic acid also inhibit enzymatic activity, amino acid uptake, (inducing cell membrane-damage) and synthesis of RNA and/or DNA; caprylic acid integrates with the cell wall due to its lipophilic character and leads to subsequent cell leaking.

The synergistic antimicrobial activity of compositions of the present invention may provide an improved treatment of pathogenic microorganisms and may play an important role in fighting the growing problem of antibiotic resistance.

Antimicrobial compositions of the present invention may kill and/or inhibit the growth of microorganisms including bacteria, fungi, algae, protozoa, viruses and sub-viral agents. The compositions may be microbicidal or microbiostatic and can be disinfectants, antiseptics or antibiotics. Antimicrobial compositions of the invention may be antibacterial, antifungal or antiparasitic.

Garlic, also known as *Allium sativum,* is a species in the onion genus. Garlic extracts used in the present invention can include one or more of crude garlic, allicin, ajoene or a combination thereof. The garlic extracts can also be diallyl disulphide (DADS) or S-allyl cysteine (SAC). Crude garlic may be, for example, in a fresh or crushed form and encompasses garlic juice, pulp, infusion, cutting, distillate, residue, pressing or pomace.

Allicin (2-Propene-1-sulfinothioic acid S-2-propenyl) is a highly chemically reactive sulphur-containing compound derived from garlic and other *Allium* species. Allicin is not found natively within garlic but it is produced when the cells are damaged and the enzyme alliinase (alliin lyase) is released from the vacuoles, coming into contact with the compound alliin ((2R)-2-amino-3-[(S)-prop-2-enylsulfinyl]propanoic acid) and converting it in to allicin. Suitable allicin for use in compositions of the present invention may be obtained from a natural, synthetic or semi-synthetic source.

Ajoene ((E,Z)-4,5,9-trithiadodeca-1,6,11-triene 9-oxide), is another sulphur based compound, which can form two different isomers (E & Z). It may be used in the present invention as a mixture of both isomers or may be pure E- or Z-ajoene. Ajoene is derived from the thermal degradation of allicin under specific conditions.

Compositions of the invention comprise at least one garlic extract, at least one bioflavonoid and optionally an organic acid for use in treating or preventing a *Staphylococcus aureus* or *Candida tropicalis* infection, wherein the garlic extract is selected from allicin and ajoene, wherein the bioflavonoid is selected from one or more of the group consisting of naringenin, naringin, quercetin and rhoifolin, and wherein the organic acid is a carboxylic acid.

Compositions of the invention may comprise particular ratios of components. For example, the ratio of garlic extract to bioflavonoid may be about 1-16:1-16, preferably 1-4:1-4. Similarly, the ratio of garlic extract to organic acid may be about 1-16:1-16, preferably 1-4:1-4. In an embodiment of the invention, the ratio of the garlic extract to the bioflavonoid and to the organic acid may be about 1-16:1-16:1-16, preferably, about 1-4:1-4:1-4. One example of composition among garlic extract, bioflavonoid and organic acid has a concentration ratio of 1: 4: 2.

Bioflavonoids (also known as flavonoids) are naturally occurring polyphenol compounds produced by plants. Chemically, bioflavonoids have the structure of a 15-carbon skeleton, consisting of two phenyl rings and a heterocyclic ring and are subdivided into categories based on their basic structure. Bioflavonoids include anthoxanthins, such as flavones, flavonols, flavanones (including flavanone-glycosides), flavanonols, flavan, anthocyanidins or any combination thereof. Alternatively, the bioflavonoids can also be isoflavonoids or neoflavonoids.

Flavones include acacetin, rhoifolin (apigenin 7-O-neohesperidoside), luteolin, apigenin and tangeritin. Flavonols include quercetin, kaempferol, myricetin, fisetin, galangin, isohamnetin, pachypodol, rhamnazin, pyranoflavonols and furanoflavonols. Flavanones include hesperetin, naringenin, eriodictyol and homoeriodictyol, as well as the flavanone-glycosides such as naringin, hesperedin and neohesperidin.

Compositions of the present invention comprise one or more of rhoifolin quercetin, naringenin, and naringin, or a combination thereof.

Compositions described herein may comprise allicin with one or more of a flavone, a flavonol, a flavanone, a flavanonol, a flavan and an anthocyanidin, or a combination thereof.

Alternatively, compositions described herein may comprise ajoene with one or more of a flavone, a flavonol and a flavanone, a flavanonol, a flavan and an anthocyanidin, or a combination thereof. The compositions may additionally include one or more organic acids.

Organic acids are organic compounds having acidic properties and include carboxylic acids and sulfonic acids. Suitable organic acids for use in compositions of the present invention include, but are not limited to, lactic acid, butyric acid, propionic acid, valeric acid, caproic acid, acetic acid, formic acid, citric acid, oxalic acid, sorbic acid, benzoic acid, caprylic acid and malic acid, or any combination thereof.

Compositions of the present invention may additionally include one or more pharmaceutically acceptable carriers and/or excipients, such as diluents, adjuvants, excipients, vehicles, fillers, binders, disintegrating agents, wetting agents, emulsifying agents, suspending agents, perfuming agents, buffers, dispersants, thickeners, solubilising agents, lubricating agents and dispersing agents, depending on the nature of the mode of administration and dosage forms.

The compositions may take the form, for example, of solid preparations including tablets, capsules, drageés, lozenges, granules, powders, pellets and cachets; and liquid preparations including gels, lotions, suspensions, elixirs, syrups, suspensions, sprays, emulsions and solutions.

The composition of the present invention may be administered in the form of a composition comprising any suitable additional component, such as an additional antimicrobial agent, a nutraceutical, or a dietary supplement.

The composition of the present invention can also contain an additive, such as flavouring agents, colourants, stabilizers, preservatives, artificial and natural sweeteners and the like.

Compositions described herein may be for use in treating or preventing a bacterial infection. The bacterial infection may be a Gram-positive bacterial infection or a Gram-negative bacterial infection, or a combination thereof. Gram-positive bacteria include, for example, *Streptococci*, such as S. *viridans, Staphylococci,* such as *S*. *aureus,* and *Bacillus,* such as *B. subtilis, B. anthracis and B. cereus.*

Gram-negative bacteria include, for example, *E. coli, Pseudomonas,* such as *P. aeruginosa,* and *Klebsiella,* such as *K. pneumonia, K. aerogenes* and *K. oxytoca.* Preferably, the infection is a Gram-negative bacterial infection. The composition can also be for use in treating or preventing Gram-positive bacterial infection.

Compositions described herein may be for use in treating or preventing a fungal infection. The fungal infection may be a *Candida* infection, for example, an infection with *C*. *albicans, C. parapsilosis* or *C*. *tropicalis,* or a combination thereof.

The composition may be formulated for oral, topical, intravenous, intramuscular, intrarectal (suppository), transdermal, sublingual, subcutaneous or intranasal administration. Preferably the composition is formulated for oral or topical administration.

The composition can be combined with a food or foodstuff before oral consumption. The solid or liquid form preparations may be mixed into the food or foodstuff or applied to the food, foodstuff or feed of a subject. Such solid forms include powders, granules, pellets and the like.

The subject may be a human, a primate, bovine, ovine, equine, porcine, avian, rodent (such as mouse or rat), feline, or canine. Preferably, the subject is a human. The subject can also be production animals such as cattle, oxen, deer, goats, sheep and pigs, working and sporting animals such as dogs, horses and ponies, companion animals such as dogs and cats, and laboratory animals such as rabbits, rats, mice, hamsters, gerbils or guinea pigs.

Also disclosed herein is the use of an antimicrobial composition comprising at least one garlic extract and one or more of a bioflavonoid and/or an organic acid in the manufacture of a medicament for treating or preventing a bacterial infection in a subject, or in the manufacture of a medicament for treating or preventing a fungal infection in a subject, or in the manufacture of a medicament for treating or preventing a parasitic infection in a subject.

The antimicrobial composition as described herein can be useful as a treatment or prophylaxis against common pathological microorganisms, especially those widely distributed and can be commensals of the body of human or mammals. These microorganisms can include the pathogens present in the human or mammals' upper respiratory tracts, gastrointestinal tracts, oral cavities, skin, urinary tracts, or female genital tracts.

### Brief Description of the Drawings

The invention is now described in specific embodiments with reference to accompanying drawings in which:
Figure 1 shows the response of Gram-positive bacteria (*B. subtilis, S. aureus and S. viridans*) (1A), Gram-negative bacteria (*E. coli, P. aeruginosa and K. pneumonia*) (1B) and yeast (C. *tropicalis)* (1C) to allicin, as well as the response of bacteria (*B. subtilis, S. aureus, S. viridans, E. coli, P. aeruginosa and K. pneumonia)* (1D) and yeast *(C. tropicalis)* (1E) to ajoene.

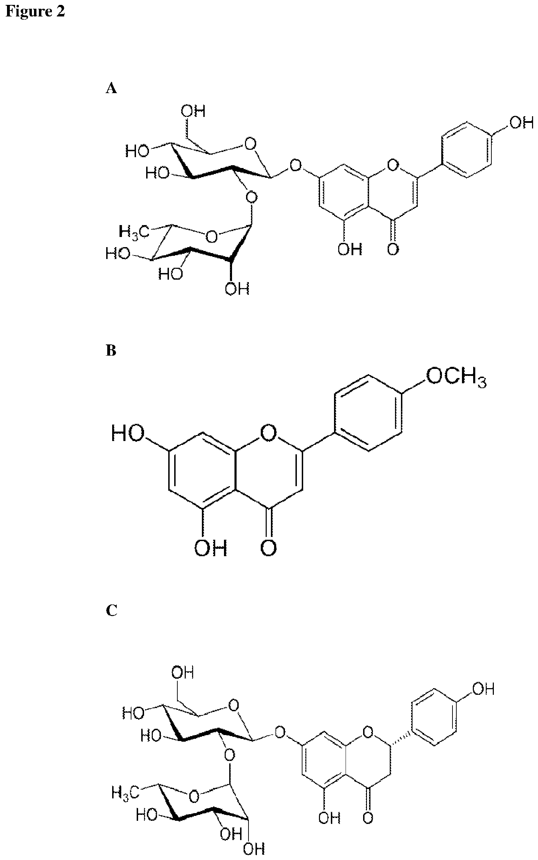

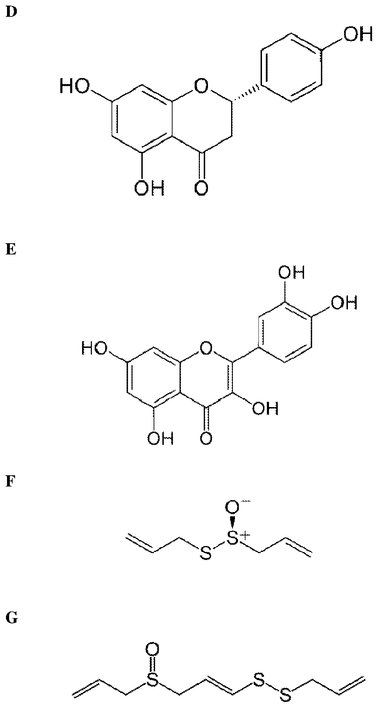

Figure 2 shows the structures of rhoifolin (A), acacetin (B), naringin (C), naringenin (D), quercetin (E), allicin (F) and ajoene (G).

### Examples

Examples are provided below to illustrate different aspects and embodiments of the present invention. These examples are not intended in any way to limit the disclosed invention, which is limited only by the claims.

### Example 1 Synergistic Test between Garlic Extracts and Bioflavonoids

A synergistic test was conducted to test whether the antimicrobial efficacy of allicin and ajoene can be enhanced synergistically by polyphenols in the flavone (such as rhoifolin), flavonol (such as quercetin), flavanone (such as naringenin) and flavanone glycoside (such as naringin) groups of bioflavonoids.

The presence of synergy was determined by subtracting individual inhibition results for each compound from a mixed inhibition result for the corresponding compounds and concentrations. Any result greater than 0 was considered to be synergistic because the combination has a greater effect than the sum of its individual parts.

In order to determine the minimal inhibitory concentration (MIC) for the bioflavonoids (BC), allicin (AL) and ajoene (AJ) on selected Gram-positive bacteria, Gram-negative bacteria and yeast, a modified broth dilution assay using 96-well plates, according to Wiegand, Hilpert and Hancock (2008), was performed.

### Example 2 Preparation of Microbial Cultures

For testing purposes, a Gram-positive species (S. *aureus*), three Gram-negative species (*E. coli, P. aeruginosa and K. pneumonia*) and a yeast (C. *tropicalis)* were used.

The microorganisms for the study were obtained from Cardiff University. The cultures used in the study were prepared by aseptic inoculation of microorganisms from pure plate cultures into 50 ml of sterile nutrient broth. The cultures were incubated overnight at 37.0°C without agitation. Prior to use the cultures were washed by centrifugation in a desk top centrifuge at 3000 RPM for 15 minutes and the pellet resuspended in sterile saline (0.85% NaCl).

### Example 3 Preparation of Compounds

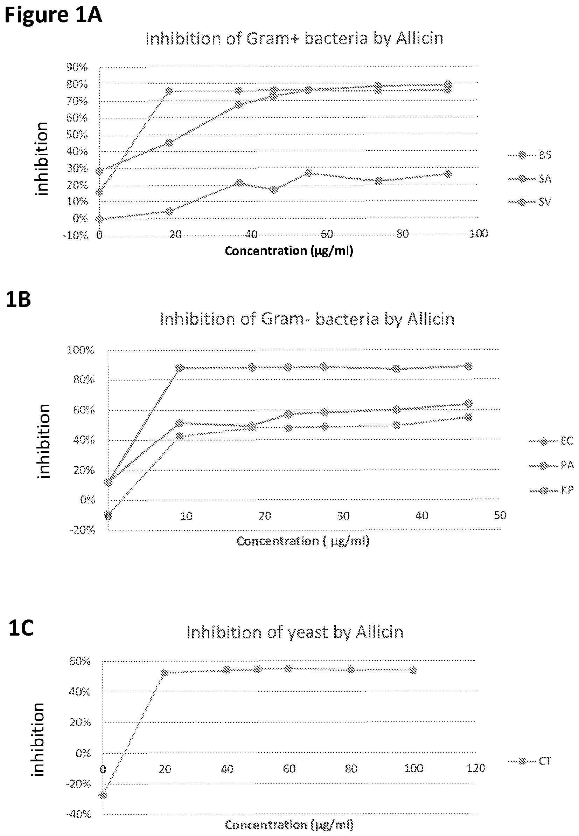

In order to determine the activity of the compounds both individually as well as in combination, a range of concentrations of each compound was used in a doubling dilution. The highest concentration used was dependant on the organism in question (whether Gram-positive bacteria, Gram-negative bacteria or yeast). From the highest concentration used in the test the samples were diluted five times in order to give six different concentrations per compound. The compounds were all tested individually as well as in a matrix of concentrations including mixtures of AL:BC, AJ:BC and AL:AJ:BC. The dilution gradients of AL and AJ were run inversely to BC (as shown in Figure 1A-H).

The identities of the garlic compounds were confirmed by HPLC analysis using a calibrated method. The Bioflavonoids were purchased from Sigma-Aldrich and were supplied with Certificates of Analysis to confirm their identity.

The allicin samples were aqueous solutions and were therefore diluted directly using H₂O. However, the ajoene was in oil form and the bioflavonoids were in powder form, neither of which were water soluble. Before dilution the ajoene and bioflavonoids were solubilised by creation of a stock solution using 80% DMSO.

### Example 4 Preparation of Plates

The wells on the 96-well plates were prepared in triplicate as follows: the AL, AJ and BC were pre-diluted in Eppendorf tubes at 10 x the required final concentration. 20µl of each dilution was added per well to give a 1:10 dilution in the final 200 µl per well. A matrix of concentrations of mixtures of allicin:bioflavonoid, ajoene:bioflavonoid and allicin:ajoene:bioflavonoid was used, with the dilution gradients of allicin and ajoene running inversely to bioflavonoids. The concentrations used are 50 µg/ml, 25 µg/ml, 12.5 µg/ml, 6.25 µg/ml, 3.125 µg/ml and 1.5625 µg/ml.

130µl of sterile nutrient broth (Nutrient Broth No. 3 (Sigma 70149)) was added to each well.

50µl of each microorganism suspended in saline was added to each well (except for the negative control). Each species of microorganism was added to two plates, the first for the individual (control) assays and the second for the matrix study. The plates were incubated at 37.0°C overnight without agitation.

### Example 5 Data Collection and Analysis

The plates were scanned individually using a Tecan plate reader at 600nm and the absorbance recorded in a comma-delimited file for analysis.

The absorbance for each sample was compared to the growth (absorbance) of the control cultures (without treatment) in order to determine the MIC (all values blanked against negative control to negate absorbance of plate and broth). The mean and standard deviation of the triplicate experiments were determined and the means plotted on scatter diagrams for comparison.

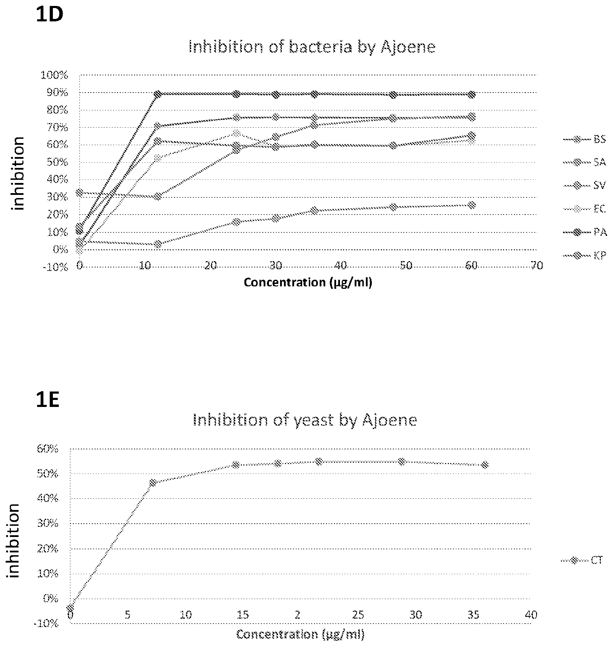

The response of the microorganisms to the individual compounds of garlic extracts was dose (concentration) dependent as seen in Figure 1. The MIC was reached at very low concentrations as reflected in Table 1.

**Table 1 MICs for AL and AJ as determined in one example of the studies:**

| **Organism** | **Allicin** | **Ajoene** |
|---|---|---|
| *Bacillus subtilis* | 9.2µg/ml | 24µg/ml |
| *Staphylococcus aureus* | 27.6µg/ml | 48µg/ml |
| *Escherichia coli* | 18.4µg/ml | 24µ/ml |
| *Pseudomonas aeruginosa* | 9.2µg/ml | 12µg/ml |
| *Klebsiella pneumonia* | 46µg/ml | 12µg/ml |
| *Candida tropicalis* | 9.2µg/ml | 24µg/ml |

Synergy in antimicrobial activity was determined as any antimicrobial activity that was greater than the additive effect of the individual compounds.

Table 2 shows the presence or absence of synergy in one or more of the concentration combinations between AL, AJ, and the selected bioflavonoids. A full list of concentrations which produced synergy can be found in Table 3 for different combinations between bioflavonoids and garlic extracts. Where synergy was indicated the additive inhibitory effect of the two individual compounds was exceeded by the inhibitory effect expressed by the combination, for each combination this effect is concentration dependent.

**Table 2**

| **Garlic Extract** | **Allicin** | | | | **Ajoene** | | | |
|---|---|---|---|---|---|---|---|---|
| **Bioflavonoid** | **Naringenin** | **Naringin** | **Quercetin** | **Rhoifolin** | **Naringenin** | **Naringin** | **Quercetin** | **Rhoifolin** |
| *S. Aureus* | Yes | Yes | Yes | Yes | Yes | Yes | Yes | Yes |
| *E. coli* | Yes | Yes | Yes | Yes | Yes | Yes | Yes | Yes |
| *P. aeruginosa* | Yes | Yes | Yes | Yes | Yes | Yes | Yes | Yes |
| *K. pneumoniae* | Yes | Yes | Yes | Yes | Yes | Yes | Yes | Yes |
| *C. tropicalis* | Yes | Yes | Yes | Yes | Yes | Yes | Yes | Yes |

Table 3 shows the synergy determination for different concentrations of allicin and ajoene, respectively, in combination with four individual bioflavonoids (naringenin, naringin, quercetin and rhoifolin). In Table 3, the results are presented in the sequence allicin or ajoene + Bioflavonoid.

**Table 3**

| **Garlic Extract** | **Allicin (µg/ml)** | | | | **Ajoene (µg/ml)** | | | |
|---|---|---|---|---|---|---|---|---|
| **Bioflavonoid** | **Naringenin (µg/ml)** | **Naringin (µg/ml)** | **Quercetin (µg/ml)** | **Rhoifolin (µg/ml)** | **Naringenin (µg/ml)** | **Naringin (µg/ml)** | **Quercetin (µg/ml)** | **Rhoifolin (µg/ml)** |
| *S. Aureus* | 25 + 1.5625 | 6.25+6.25, 12.5+3.125, 25+1.5625 | 3.125+12.5, 6.25+6.25 | 1.5625+25, 3.125+12.5, 6.25+6.25, 12.5+3.125, 25+1.5625 | 12.5+3.125, 25+1.5625 | 3.125+12.5, 6.25+6.25, 12.5+3.125, 25+1.5625 | 3.125+12.5, 6.25+6.25 | 1.5625+25, 3.125+12.5, 6.25+6.25, 12.5+3.125, 25+1.5625 |
| *E. coli* | 1.5625+25, 3.125+12.5, 6.25+6.25, 12.5+3.125, 25+1.5625 | 1.5625+25, 3.125+12.5, 6.25+6.25, 12.5+3.125, 25+1.5625 | 1.5625+25, 3.125+12.5, 6.25+6.25, 12.5+3.125, 25+1.5625 | 1.5625+25, 3.125+12.5, 6.25+6.25, 12.5+3.125, 25+1.5625 | 1.5625+25, 3.125+12.5, 6.25+6.25, 12.5+3.125 | 1.5625+25, 3.125+12.5, 6.25+6.25, 12.5+3.125, 25+1.5625 | 1.5625+25, 3.125+12.5, 6.25+6.25, 12.5+3.125, 25+1.5625 | 1.5625+25, 3.125+12.5, 6.25+6.25, 12.5+3.125 |
| *P. aeruginosa* | 1.5625+25, 3.125+12.5 | 1.5625+25, 3.125+12.5, 12.5+3.125, 25+1.5625 | 1.5625+25, 3.125+12.5, 6.25+6.25, 12.5+3.125, 25+1.5625 | 1.5625+25, 3.125+12.5, 6.25+6.25, 12.5+3.125, 25+1.5625 | 6.25+6.25 | 6.25+6.25 | 1.5625+25, 3.125+12.5, 6.25+6.25, 12.5+3.125 | 1.5625+25, 3.125+12.5, 6.25+6.25, 12.5+3.125, 25+1.5625 |
| *K. pneumoniae* | 1.5625+25, 3.125+12.5, 6.25+6.25 | 1.5625+25, 3.125+12.5, 6.25+6.25, 12.5+3.125 | 1.5625+25, 3.125+12.5, 6.25+6.25, 12.5+3.125 | 6.25+6.25, 12.5+3.125 | 1.5625+25, 3.125+12.5, 6.25+6.25, 12.5+3.125 | 1.5625+25, 3.125+12.5, 6.25+6.25, 12.5+3.125 | 1.5625+25, 3.125+12.5, 6.25+6.25, 12.5+3.125, 25+1.5625 | 1.5625+25, 3.125+12.5, 6.25+6.25, 12.5+3.125 |
| *C. tropicalis* | 25+1.5625 | 25+1.5625 | 3.125+12.5, 6.25+6.25, 12.5+3.125 | 3.125+12.5, 6.25+6.25, 12.5+3.125, 25+1.5625 | 12.5+3.125, 25+1.5625 | 1.5625+25, 3.125+12.5, 6.25+6.25, 12.5+3.125, 25+1.5625 | 1.5625+25, 3.125+12.5, 6.25+6.25, 12.5+3.125, 25+1.5625 | 12.5+3.125, 25+1.5625 |

Synergy can be seen in the 20 test parameters with allicin as well as the 20 test parameters with ajoene. This shows a high synergy rate between garlic and bioflavonoids for the organisms tested, in particular with the Gram-negative organisms selected.

Some of the synergistic combinations are more concentration dependant than others, for example as seen in Table 3, *C*. *tropicalis* only showed a synergistic effect at one concentration for both allicin + naringin and allicin + naringenin. In contrast, *E. coli* showed a synergistic effect across all concentration ranges for all bioflavonoids when combined with allicin.

The results of this study suggest that synergy between alternatives may be a good place to look for a solution to the growing problem of antibiotic resistance. The results of this study may provide an answer to this threat as the synergistic effects were particularly high against the Gram-negative organisms tested.

### Example 6 Synergistic Test among Garlic Extracts, Bioflavonoids and Organic Acids

An experimental matrix was set up to study the synergism among garlic extracts, bioflavonoids and organic acids. The exemplary compounds selected were:
(a) garlic extracts: allicin (Al) and ajoene (Aj);
(b) bioflavonoids: quercetin (Q) and acacetin (Ac); and
(c) organic acids: citric acid (C) and malic acid (M).

The preparation of microbial cultures, compounds, plates were conducted using the procedures as detailed in Examples 2 to 4. Data was collected and analysed as shown in Tables 4 and 5.

Table 4 shows a summary of the absence or presence of synergistic inhibitory effects of different combinations among garlic extracts, bioflavonoids and organic acids against the similar series of microorganisms as stated in Example 2, i.e. *S*. *aureus*, *E. coli, P. aeruginosa, K. pneumonia*, *C. tropicalis*, with an additional strain of Gram-positive bacteria *S*. *viridans.* Table 5 is a full list of concentrations which produced synergy. In Table 5, the results in concentration ratio are presented in the sequence of (allicin or ajoene) + (malic acid or citric acid) + (acacetin or quercetin).

There is a high rate of synergy shown by the combinations of the three active ingredients, i.e. garlic extract, bioflavonoid and organic acid, with respect to the organisms tested, which include Gram-positive bacteria (*S. aureus* and *S*. *viridans*), Gram-negative bacteria (*E. coli, P. aeruginosa and K. pneumonia*) and yeast (C. *tropicalis).*

**Table 4**

| **Garlic Extract** | **Allicin** | | | | **Ajoene** | | | |
|---|---|---|---|---|---|---|---|---|
| **Bioflavonoid** | **Acacetin** | | **Quercetin** | | **Acacetin** | | **Quercetin** | |
| **Organic Acid** | **Malic acid** | **Citric acid** | **Malic acid** | **Citric acid** | **Malic acid** | **Citric acid** | **Malic acid** | **Citric acid** |
| *S. aureus* | Yes | Yes | Yes | Yes | Yes | Yes | Yes | Yes |
| *S. viridans* | Yes | Yes | Yes | Yes | Yes | Yes | Yes | Yes |
| *E. coli* | Yes | Yes | Yes | Yes | Yes | Yes | Yes | Yes |
| *P. aeruginosa* | Yes | Yes | Yes | Yes | Yes | Yes | Yes | Yes |
| *K. pneumoniae* | Yes | Yes | Yes | Yes | Yes | Yes | Yes | Yes |
| *C. tropicalis* | Yes | Yes | Yes | Yes | Yes | Yes | Yes | Yes |

**Table 5**

| **Garlic Extract** | **Allicin (µg/ml)** | | | | **Ajoene (µg/ml)** | | | |
|---|---|---|---|---|---|---|---|---|
| **Bioflavonoi d** | **Acacetin (µg/ml)** | | **Quercetin (µg/ml)** | | **Acacetin (µg/ml)** | | **Quercetin (µg/ml)** | |
| **Organic Acid** | **Malic acid (µg/ml)** | **Citric acid (µg/ml)** | **Malic acid (µg/ml)** | **Citric acid (µg/ml)** | **Malic acid (µg/ml)** | **Citric acid (µg/ml)** | **Malic acid (µg/ml)** | **Citric acid (µg/ml)** |
| *S. aureus* | 25+1.5625+1.562 5, 12.5+3.125+3.12 5, 6.25+6.25+6.25, 3.125+12.5+12.5, 1.5625+25+25 | 25+1.5625+1.562 5, 12.5+3.125+3.12 5, 6.25+6.25+6.25, 3.125+12.5+12.5, 1.5625+25+25 | 25+1.5625+1.562 5, 12.5+3.125+3.12 5, 6.25+6.25+6.25 | 25+1.5625+1.562 5, 12.5+3.125+3.12 5 | 25+1.5625+1.562 5, 12.5+3.125+3.12 5, 6.25+6.25+6.25, 3.125+12.5+12.5, 1.5625+25+25 | 25+1.5625+1.562 5, 12.5+3.125+3.12 5, 6.25+6.25+6.25, 3.125+12.5+12.5, 1.5625+25+25 | 25+1.5625+1.562 5, 12.5+3.125+3.12 5, 6.25+6.25+6.25, 3.125+12.5+12.5, 1.5625+25+25 | 25+1.5625+1.562 5, 12.5+3.125+3.12 5, 6.25+6.25+6.25, 3.125+12.5+12.5 |
| *S. viridans* | 25+1.5625+1.562 5, 12.5+3.125+3.12 5, 6.25+6.25+6.25, 3.125+12.5+12.5, 1.5625+25+25 | 25+1.5625+1.562 5, 12.5+3.125+3.12 5 | 25+1.5625+1.562 5, 12.5+3.125+3.12 5, 6.25+6.25+6.25 | 25+1.5625+1.562 5, 6.25+6.25+6.25 | 25+1.5625+1.562 5, 12.5+3.125+3.12 5, 6.25+6.25+6.25, 3.125+12.5+12.5, 1.5625+25+25 | 25+1.5625+1.562 5, 12.5+3.125+3.12 5, 6.25+6.25+6.25, 3.125+12.5+12.5, 1.5625+25+25 | 25+1.5625+1.562 5, 12.5+3.125+3.12 5, 6.25+6.25+6.25, 3.125+12.5+12.5, 1.5625+25+25 | 25+1.5625+1.562 5, 12.5+3.125+3.12 5, 6.25+6.25+6.25, 3.125+12.5+12.5, 1.5625+25+25 |
| *E. coli* | 12.5+3.125+3.12 5, 6.25+6.25+6.25, 3.125+12.5+12.5, 1.5625+25+25 | 12.5+3.125+3.12 5, 6.25+6.25+6.25, 3.125+12.5+12.5, 1.5625+25+25 | 25+1.5625+1.562 5, 12.5+3.125+3.12 5, 6.25+6.25+6.25, 3.125+12.5+12.5, 1.5625+25+25 | 25+1.5625+1.562 5, 12.5+3.125+3.12 5, 6.25+6.25+6.25, 3.125+12.5+12.5, 1.5625+25+25 | 25+1.5625+1.562 5, 12.5+3.125+3.12 5, 6.25+6.25+6.25, 3.125+12.5+12.5, 1.5625+25+25 | 25+1.5625+1.562 5, 12.5+3.125+3.12 5, 6.25+6.25+6.25, 3.125+12.5+12.5, 1.5625+25+25 | 25+1.5625+1.562 5, 12.5+3.125+3.12 5, 6.25+6.25+6.25, 3.125+12.5+12.5, 1.5625+25+25 | 25+1.5625+1.562 5, 12.5+3.125+3.12 5, 6.25+6.25+6.25, 3.125+12.5+12.5, 1.5625+25+25 |
| *P. aeruginosa* | 25+1.5625+1.562 5, 12.5+3.125+3.12 5, 6.25+6.25+6.25 | 25+1.5625+1.562 5 | 25+1.5625+1.562 5, 12.5+3.125+3.12 5, 1.5625+25+25 | 25+1.5625+1.562 5, 12.5+3.125+3.12 5 | 25+1.5625+1.562 5, 12.5+3.125+3.12 5, 6.25+6.25+6.25, 3.125+12.5+12.5 | 6.25+6.25+6.25 | 25+1.5625+1.562 5, 12.5+3.125+3.12 5, 6.25+6.25+6.25, 3.125+12.5+12.5 | 12.5+3.125+3.12 5, 6.25+6.25+6.25 |
| *K. pneumoniae* | 25+1.5625+1.562 5, 12.5+3.125+3.12 5, 6.25+6.25+6.25, 3.125+12.5+12.5, 1.5625+25+25 | 25+1.5625+1.562 5, 12.5+3.125+3.12 5, 6.25+6.25+6.25, 3.125+12.5+12.5, 1.5625+25+25 | 25+1.5625+1.562 5, 12.5+3.125+3.12 5, 6.25+6.25+6.25, 3.125+12.5+12.5, 1.5625+25+25 | 25+1.5625+1.562 5, 6.25+6.25+6.25, 3.125+12.5+12.5, 1.5625+25+25 | 25+1.5625+1.562 5, 12.5+3.125+3.12 5, 6.25+6.25+6.25, 3.125+12.5+12.5, 1.5625+25+25 | 25+1.5625+1.562 5, 12.5+3.125+3.12 5, 6.25+6.25+6.25, 3.125+12.5+12.5, 1.5625+25+25 | 25+1.5625+1.562 5, 12.5+3.125+3.12 5, 6.25+6.25+6.25, 3.125+12.5+12.5, 1.5625+25+25 | 25+1.5625+1.562 5, 12.5+3.125+3.12 5, 6.25+6.25+6.25, 3.125+12.5+12.5, 1.5625+25+25 |
| *C. tropicalis* | 25+1.5625+1.562 5, 12.5+3.125+3.12 5, 6.25+6.25+6.25, 3.125+12.5+12.5, 1.5625+25+25 | 25+1.5625+1.562 5, 12.5+3.125+3.12 5, 6.25+6.25+6.25, 3.125+12.5+12.5, 1.5625+25+25 | 25+1.5625+1.562 5, 12.5+3.125+3.12 5, 6.25+6.25+6.25, 3.125+12.5+12.5 | 25+1.5625+1.562 5, 12.5+3.125+3.12 5, 6.25+6.25+6.25, 3.125+12.5+12.5, 1.5625+25+25 | 25+1.5625+1.562 5, 12.5+3.125+3.12 5, 6.25+6.25+6.25, 3.125+12.5+12.5, 1.5625+25+25 | 25+1.5625+1.562 5, 12.5+3.125+3.12 5, 6.25+6.25+6.25, 3.125+12.5+12.5, 1.5625+25+25 | 25+1.5625+1.562 5, 12.5+3.125+3.12 5, 6.25+6.25+6.25, 3.125+12.5+12.5, 1.5625+25+25 | 25+1.5625+1.562 5, 12.5+3.125+3.12 5, 6.25+6.25+6.25, 3.125+12.5+12.5, 1.5625+25+25 |

## Claims

1. An antimicrobial composition comprising:
at least one garlic extract; and
at least one bioflavonoid and optionally an organic acid for use in treating or preventing a *Staphylococcus aureus or Candida tropicalis* infection, wherein the garlic extract is selected from allicin and ajoene, wherein the bioflavonoid is selected from one or more of the group consisting of naringenin, naringin, quercetin and rhoifolin, and wherein the organic acid is a carboxylic acid.

2. An antimicrobial composition for use according to claim 1, wherein the concentration ratio of the garlic extract to the bioflavonoid is about 1-16:16-1.

3. An antimicrobial composition for use according to claim 2, wherein the concentration ratio of the garlic extract to the bioflavonoid is about 1-4:4-1.

4. An antimicrobial composition for use according to claim 1, wherein the composition comprises at least one garlic extract and at least one organic acid, preferably wherein the concentration ratio of the garlic extract to the organic acid is about 1-16:16-1.

5. An antimicrobial composition for use according to claim 4, wherein the concentration ratio of the garlic extract to the organic acid is about 1-4:4-1.

6. An antimicrobial composition for use according to claim 1, wherein the composition comprises at least one garlic extract, at least one bioflavonoid and at least one organic acid, preferably wherein the concentration ratio of the garlic extract to the bioflavonoid and to the organic acid is about 1-16:1-16:1-16.

7. An antimicrobial composition for use according to claim 6, wherein the concentration ratio of the garlic extract to the bioflavonoid and to the organic acid is about 1-4:1-4:1-4.

8. An antimicrobial composition for use according to any one of claims 1 to 7, wherein the organic acid is selected from one or more of lactic acid, butyric acid, propionic acid, valeric acid, caproic acid, acetic acid, formic acid, citric acid, oxalic acid, sorbic acid, benzoic acid, caprylic acid and malic acid, or any combination thereof.

9. An antimicrobial composition for use according to any one of claims 1 to 8 further comprising a pharmaceutically acceptable carrier and/or excipient, and/or wherein the composition is formulated for oral, topical, intravenous, intramuscular, intrarectal (suppository), inhalant, infusion, transdermal, sublingual, subcutaneous or intranasal administration.

## Patentansprüche

1. Antimikrobielle Zusammensetzung, umfassend:
mindestens einen Knoblauchextrakt; und
mindestens ein Bioflavonoid und optional eine organische Säure zur Verwendung beim Behandeln oder Vorbeugen einer Infektion mit *Staphylococcus aureus* oder *Candida tropicalis,* wobei der Knoblauchextrakt ausgewählt ist aus Allicin und Ajoen, wobei das Bioflavonoid ausgewählt ist aus einem oder mehreren aus der Gruppe bestehend aus Naringenin, Naringin, Quercetin und Rhoifolin, und wobei die organische Säure eine Carbonsäure ist.

2. Antimikrobielle Zusammensetzung zur Verwendung nach Anspruch 1, wobei das Konzentrationsverhältnis des Knoblauchextrakts zu dem Bioflavonoid etwa 1-16:16-1 beträgt.

3. Antimikrobielle Zusammensetzung zur Verwendung nach Anspruch 2, wobei das Konzentrationsverhältnis des Knoblauchextrakts zu dem Bioflavonoid etwa 1-4:4-1 beträgt.

4. Antimikrobielle Zusammensetzung zur Verwendung nach Anspruch 1, wobei die Zusammensetzung mindestens einen Knoblauchextrakt und mindestens eine organische Säure umfasst, wobei vorzugsweise das Konzentrationsverhältnis des Knoblauchextrakts zu der organischen Säure etwa 1-16:16-1 beträgt.

5. Antimikrobielle Zusammensetzung zur Verwendung nach Anspruch 4, wobei das Konzentrationsverhältnis des Knoblauchextrakts zu der organischen Säure etwa 1-4:4-1 beträgt.

6. Antimikrobielle Zusammensetzung zur Verwendung nach Anspruch 1, wobei die Zusammensetzung mindestens einen Knoblauchextrakt, mindestens ein Bioflavonoid und mindestens eine organische Säure umfasst, wobei vorzugsweise das Konzentrationsverhältnis des Knoblauchextrakts zu dem Bioflavonoid und zu der organischen Säure etwa 1-16:1-16:1-16 beträgt.

7. Antimikrobielle Zusammensetzung zur Verwendung nach Anspruch 6, wobei das Konzentrationsverhältnis des Knoblauchextrakts zu dem Bioflavonoid und zu der organischen Säure etwa 1-4:1-4:1-4 beträgt.

8. Antimikrobielle Zusammensetzung zur Verwendung nach einem der Ansprüche 1 bis 7, wobei die organische Säure ausgewählt ist aus einer oder mehreren von Milchsäure, Buttersäure, Propionsäure, Valeriansäure, Capronsäure, Essigsäure, Ameisensäure, Zitronensäure, Oxalsäure, Sorbinsäure, Benzoesäure, Caprylsäure und Apfelsäure oder einer beliebigen Kombination davon.

9. Antimikrobielle Zusammensetzung zur Verwendung nach einem der Ansprüche 1 bis 8, ferner umfassend einen pharmazeutisch verträglichen Träger und/oder Exzipienten, und/oder wobei die Zusammensetzung zur oralen, topischen, intravenösen, intramuskulären, intrarektalen (Zäpfchen) Inhalations-, Infusions-, transdermalen, sublingualen, subkutanen oder intranasalen Verabreichung formuliert ist.

## Revendications

1. Composition antimicrobienne comprenant :
au moins un extrait d'ail ; et
au moins un bioflavonoïde et éventuellement un acide organique pour une utilisation dans le traitement ou la prévention d'une infection à *Staphylococcus aureus ou Candida tropicalis,* dans laquelle l'extrait d'ail est choisi parmi l'allicine et l'ajoène, dans laquelle le bioflavonoïde est choisi parmi un ou plusieurs du groupe consistant en naringénine, la naringine, la quercétine et la rhoifoline, et dans laquelle l'acide organique est un acide carboxylique.

2. Composition antimicrobienne à utiliser selon la revendication 1, dans laquelle le rapport de concentration de l'extrait d'ail au bioflavonoïde est d'environ 1-16:16-1.

3. Composition antimicrobienne à utiliser selon la revendication 2, dans laquelle le rapport de concentration de l'extrait d'ail au bioflavonoïde est d'environ 1-4:4-1.

4. Composition antimicrobienne à utiliser selon la revendication 1, dans laquelle la composition comprend au moins un extrait d'ail et au moins un acide organique, de préférence dans laquelle le rapport de concentration de l'extrait d'ail à l'acide organique est d'environ 1-16:16-1.

5. Composition antimicrobienne à utiliser selon la revendication 4, dans laquelle le rapport de concentration de l'extrait d'ail à l'acide organique est d'environ 1-4:4-1.

6. Composition antimicrobienne à utiliser selon la revendication 1, dans laquelle la composition comprend au moins un extrait d'ail, au moins un bioflavonoïde et au moins un acide organique, de préférence dans laquelle le rapport de concentration de l'extrait d'ail au bioflavonoïde et à l'acide organique est d'environ 1-16: 1-16: 1-16.

7. Composition antimicrobienne à utiliser selon la revendication 6, dans laquelle le rapport de concentration de l'extrait d'ail au bioflavonoïde et à l'acide organique est d'environ 1-4:1-4:1-4.

8. Composition antimicrobienne à utiliser selon l'une quelconque des revendications 1 à 7, dans laquelle l'acide organique est choisi parmi un ou plusieurs parmi l'acide lactique, l'acide butyrique, l'acide propionique, l'acide valérique, l'acide caproïque, l'acide acétique, l'acide formique, l'acide citrique, l'acide oxalique, l'acide sorbique, l'acide benzoïque, l'acide caprylique et l'acide malique, ou leur combinaison.

9. Composition antimicrobienne à utiliser selon l'une quelconque des revendications 1 à 8, comprenant en outre un support et/ou un excipient pharmaceutiquement acceptable, et/ou dans laquelle la composition est formulée pour une administration orale, topique, intraveineuse, intramusculaire, intrarectale (suppositoire), par inhalation, par infusion, transdermique, sublinguale, sous-cutanée ou intranasale.
